# EUROPEAN PATENT APPLICATION

(11) **EP 4 790 415 A1**
(43) Date of publication of application: **12.08.2026**
(21) Application number: 24873969.0
(22) Date of filing: 02.10.2024
(51) Int. Cl.: G01N 33/50, G01N 33/68

(54) **METHOD FOR THE IN VITRO DIAGNOSIS OF CARDIOVASCULAR DISEASE**

(30) Priority: 03.10.2023 CL 202302964
(71) Applicant: Universidad de los Andes, 7550000 Santiago (CL); CLÍNICA DÁVILA, Recoleta Santiago (CL)
(72) Inventor: IRARRAZABAL MUÑOZ, Carlos Ernesto, Santiago (CL); LARREA GÓMEZ, Ricardo, Santiago (CL)
(74) Representative: Elzaburu S.L.P.
(86) International application number: PCT/CL2024/050121
(87) International publication number: WO 2025/073070

(57) **Abstract**

The present invention provides an in vitro diagnostic method of cardiovascular disease or pathophysiological states by detecting, quantifying and characterizing membrane protein biomarkers associated with extracellular vesicles present in blood plasma. The method involves the quantification of extracellular vesicles containing the hERG1 protein and/or the Hsp47 protein.

## Description

### Field of Invention

The invention relates to the *in vitro* diagnosis of cardiovascular disease or pathophysiological states through the detection, quantification and characterization of membrane protein biomarkers associated with extracellular vesicles present in peripheral blood.

The diagnostic method according to the invention may be used as a complement to the routine clinical examination and analyses determined by the treating physician.

### BACKGROUND

Cardiovascular Diseases (CVD), characterized by dysfunction of the heart or blood vessels, are the leading cause of death in the world. They are associated with common risk factors, such as smoking, reduced physical activity, diet type, overweight, high VLDL cholesterol, high blood pressure, and abnormal blood glucose.

The World Health Organization (WHO) estimates that approximately 32% of all deaths worldwide are due to CVD. Cardiac ischemia is defined as an inadequate supply of blood to the heart, also called coronary heart disease (CHD) or coronary artery disease, which is the term given to heart problems caused by narrowing of the coronary arteries that supply blood to the heart muscle. Ischemia is a self-spreading process that irreversibly impairs cardiac function and negatively affects prognosis. In addition, patients with coronary heart disease are at high risk of experiencing sudden acute myocardial infarction (AMI) and death.

After AMI, many patients develop heart failure (HF), the incidence and prevalence of which is very high worldwide. It is estimated that 1 - 2% of the population has HF, and this prevalence increases to 10% in the population aged 70 years or older, in 26 developed countries. Chronic decompensated HF (acute exacerbation of chronic HF), in which there is an acute or gradual exacerbation of the signs and symptoms of HF at rest in patients previously diagnosed with HF, requires additional and immediate therapy and is most commonly caused by low-adherence treatments, associated with water and sodium restrictions and inappropriate use of prescribed medications.

Despite advances in the treatment of cardiovascular diseases, this pathology is the leading cause of mortality worldwide, so it is necessary to develop new diagnostic and therapeutic tools.

Currently, there are numerous diagnostic tests to establish cardiovascular diseases with different sensitivity, specificity, cost, invasiveness, and patient accessibility. Current diagnostic methods consist of (i) Stress test and coronary angiography for cardiac ischemia, (ii) Troponin in blood for acute heart infarction and (iii) ProNP peptide for heart failure. All these assays are widely used around the world, however, each one has its limitations: stress testing and coronary angiography cannot be performed on all patients, troponin analysis is not precocious, or, the ProNP peptide does not allow for monitoring of the evolution of the disease.

For coronary heart disease, the most commonly used test is the cardiac stress test, which is a rapid, non-invasive test, but requires a cardiology lab and cardiologist. In a meta-analysis (n=1,575) it was established that the cardiac stress test has limited sensitivity (mean 67%; 54-78%) and specificity (mean 46%; 30-64%), with a serious problem of differences in the sex of the patient (positive predictive value: women 22% and men 48%), leaving women with fewer opportunities for a good diagnosis and treatment.

There are other tests with greater sensitivity and specificity than the cardiac stress test, but they must be performed in medical centers with specialized health personnel, mainly cardiologists, radiologists, nurses, nuclear medicine units, cardiology departments and imaging laboratories, and high-cost equipment. Additionally, some tests require that the patient does not have other pathologies such as allergies to contrast media, kidney or liver disease.

Therefore, the diagnosis of coronary heart disease with currently available tests is limited and consequently, treatment may be untimely and/or inadequate.

Small extracellular vesicles (sEVs) are vesicles contained in a membrane and are secreted by cells into the extracellular space and constitute a heterogeneous and dynamic group of lipid membrane nanoparticles. sEVs have been recognized as potent vehicles of intercellular communication due to their ability to transfer proteins, lipids, and nucleic acids, which influences various physiological functions and pathological functions. Over the past 20 years, interest in these sEVs has increased considerably due to their involvement in cell-to-cell communication, cell activation, inflammation, homeostasis, as well as disease development and progression. Numerous studies have characterized the content of sEVs, establishing that they contain a wide variety of proteins, sugars, lipids and different types of ribonucleic acids (RNA). For this reason, the role of sEVs has gained interest as promising sources of biomarkers for diagnosis, prognosis, follow-up and as treatment targets for various pathologies.

To date, there are *in vitro* studies that have described a potential protective role of sEVs in cardiomyocytes by activating membrane receptors that trigger signaling pathways involved in protection. In addition, an *in vivo* study determined the cardioprotective role of exosomes that had heat shock protein 70 (HSP70) on their surface, activating signaling pathways by binding to the toll-like receptor 4 (TLR4) and various kinases, leading to the activation of the HSP27 protein that possesses cardioprotective properties. However, despite the fact that there are certain elements of sEVs known in the state of the art, to date there are no developments that allow for the *in vitro* diagnosis of cardiovascular disease with the appropriate sensitivity and specificity for a correct diagnosis.

### BRIEF DESCRIPTION OF THE FIGURES

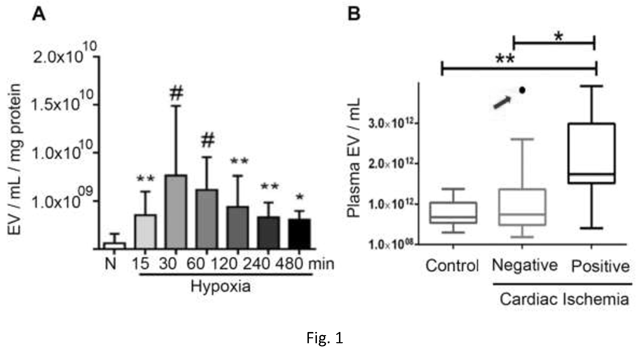

Figure 1. The decrease in oxygen concentration results in an increase in the release of sEVs from the heart. A. Cardiomyocytes in culture subjected to hypoxia (low oxygen concentration) release sEVs from 15 minutes. B Patients who experience cardiac ischemia (decrease in blood reaching the heart) release sEVs into the blood within 15 minutes.

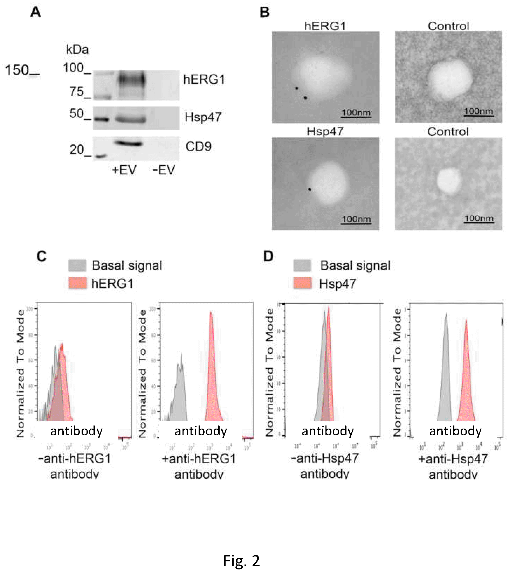

Figure 2. The proteins h-ERG1 and Hsp47 are expressed on the surface of extracellular vesicles in blood samples from patients undergoing cardiological study. A. Western blot of h-ERG1 and Hsp47 of sEVs from blood purified by ultracentrifugation was analyzed with anti-hERG1 and anti-Hsp47 antibodies. B. Transmission electron microscopy (TEM) showing the presence of h-ERG1 and Hsp47 in the membrane of sEVs that were incubated with anti-hERG1 or anti-Hsp47 antibodies. It is then incubated with gold-conjugated secondary antibody (IgG-Au). The control consisted only of incubation with sEV plus IgG-Au. C. Flow cytometry that determines the presence of h-ERG1, sEV was incubated with anti-hERG1 and anti-IgGFITC antibodies. D. Flow cytometry that determines the presence of Hsp47, sEVs were incubated with anti-Hsp47 and anti-IgGFITC antibodies.

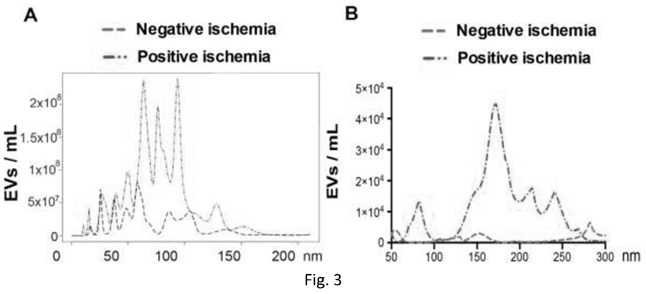

Figure 3. Extracellular vesicles containing h-ERG1 (A) or Hsp47 (B) are increased in the blood of patients with a positive diagnosis of cardiac ischemia.

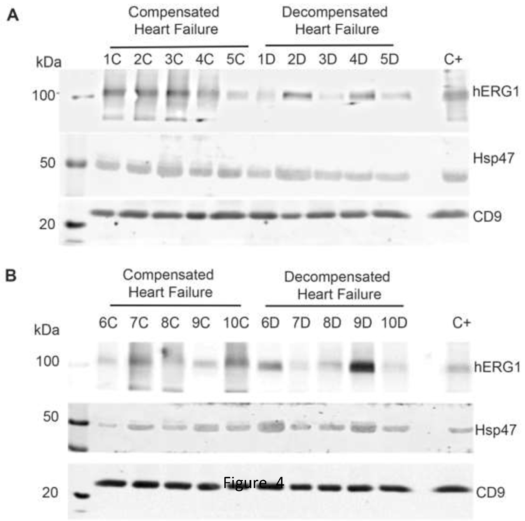

Figure 4. The proteins h-ERG1 and Hsp47 are expressed in extracellular vesicles enriched by ultracentrifugation of blood from patients with compensated heart failure and with decompensated heart failure.

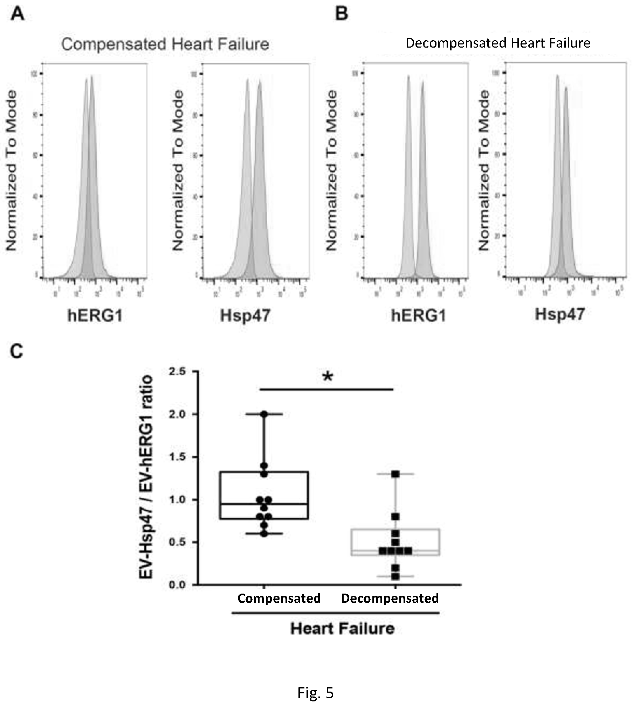

Figure 5. Expression of h-ERG1 and Hsp47 detected by flow cytometry in extracellular vesicles enriched by ultracentrifugation of blood from patients under study. A. Diagnosis of compensated heart failure. B. diagnosis of decompensated heart failure. C. Ratio of sEVs levels containing Hsp47 and h-ERG1.

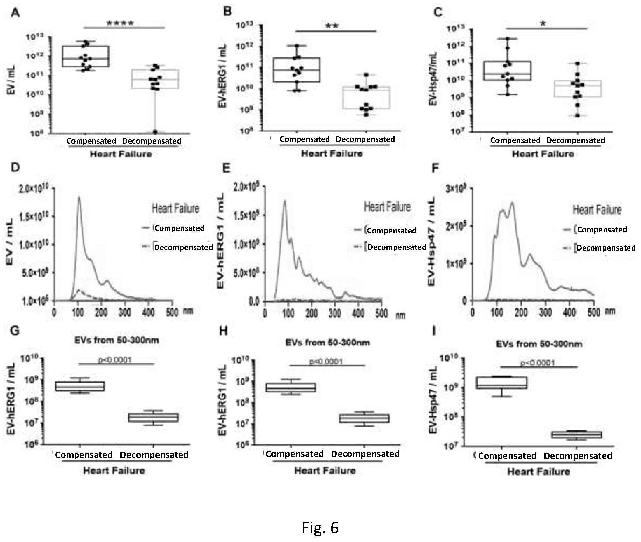

Figure 6. Concentration of total sEVs, sEVs with hERG1 (EV-hERG1) and sEVs with Hsp47 (EV-Hsp47) in patients with compensated heart failure and decompensated heart failure. A. Concentration of total sEVs. B. Concentration of sEVs with hERG1. C. Concentration of sEVs with Hsp47. D. Concentration of total sEVs of different sizes. E. Concentration of sEVs with hERG1 of different sizes. F. Concentration of sEVs with Hsp47 of different sizes. G. Concentration of sEVs of the size range of 50-300nm. H. Concentration of sEVs with hERG1 from the size range of 50-300nm. I. Concentration of sEVs with Hsp47 of the size range of 50-300nm.

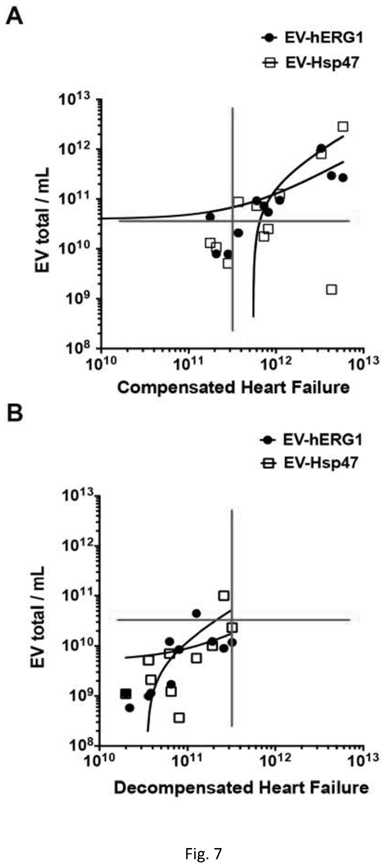

Figure 7. Correlation between total sEVs expression levels with hERG1 sEVs (EV-HERG1) or Hsp47 sEVs (EV-Hsp47). A. Patients with compensated heart failure. B. Patients with decompensated heart failure. This correlation allows us to establish a relationship to discriminate between compensated and decompensated heart failure. The y-axis represents the concentration of total sEVs. The X-axis represents blood concentrations of EV-HERG1 or EV-Hsp47.

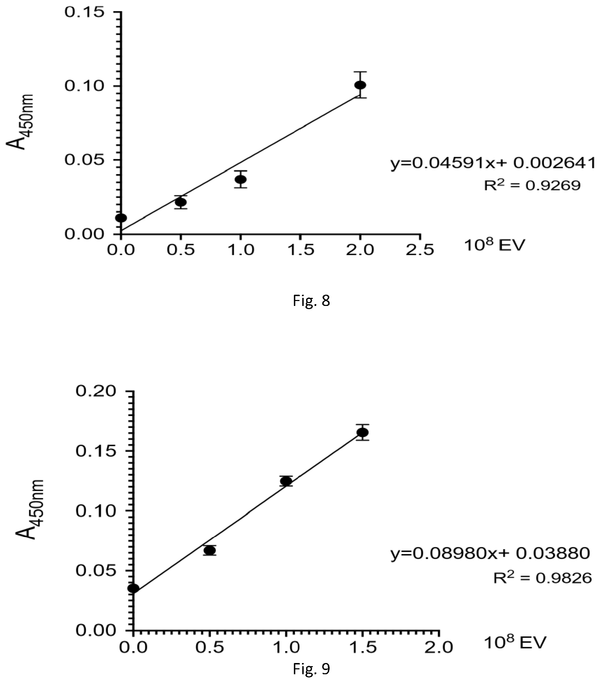

Figure 8. Standard Curve of ELISA EV ^{Hsp47+/CD63+} Prototype. This ELISA prototype purifies sEVs containing the HSP47 protein and detects sEVs purified with CD63 (EV ^{Hsp47+/CD63+}). The standard curve is made with sEVs derived from a human cardiomyocyte cell line (AC-16, distributed by Merck) subjected to hypoxia (1% O₂) and oxidative stress (hydrogen peroxide, H₂O₂ 300uM). Hypoxia is established by culturing cardiomyocytes with 1% oxygen and oxidative stress with 300uM of H₂O₂ for one hour.
Figure 9. Standard Curve of ELISA EV^{-hERG1/CD81} Prototype. This ELISA prototype purifies sEVs containing the hERG1 protein and detects sEVs purified with CD81 (EV^{-hERG1/CD81}). The standard curve is made with sEVs derived from the human cardiomyocyte cell line (AC-16, distributed by Merck) subjected to conditions of oxygen deprivation (hypoxia) and oxidative stress (hydrogen peroxide, H₂O₂). Hypoxia is established by culturing cardiomyocytes with 1% oxygen and oxidative stress with 300uM of H₂O₂ for one hour.

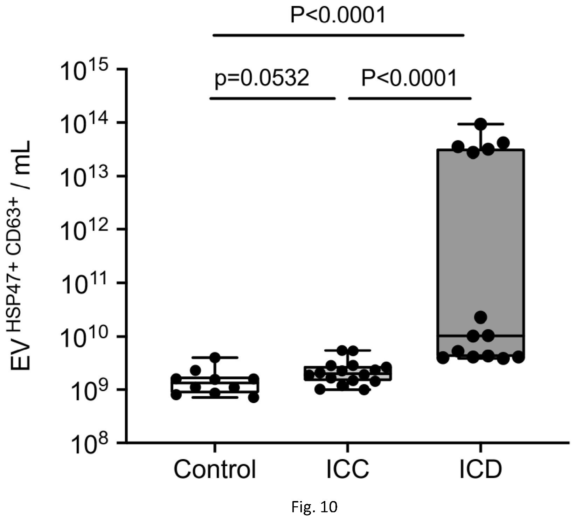

Figure 10. Concentration of EVs containing the HSP47 and CD63 proteins in patients under study. From 0.1 mL of platelet-free plasma, the concentration of EV ^{HSP47+CD63+} was determined in a healthy control group without known CVD (n=9), compensated heart failure (CHF, n=16) and decompensated heart failure (DHF, n=14). Statistical significance is indicated using a non-parametric t-test.

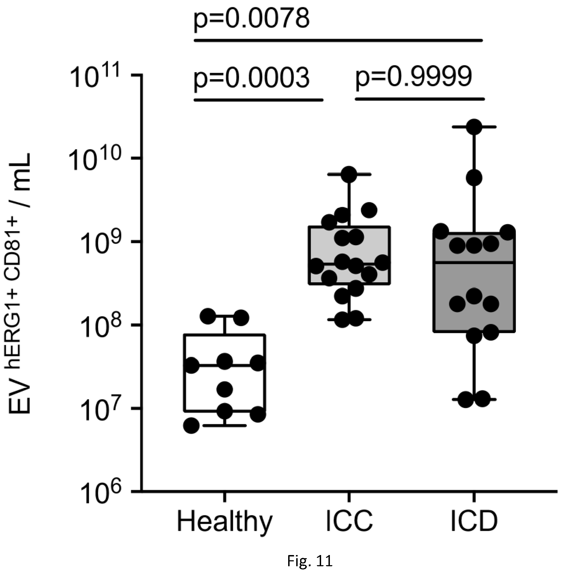

Figure 11. Concentration of EVs containing the hERG1 and CD81 proteins in patients under study. From 0.1 mL of platelet-free plasma, the concentration of EV ^{hERG1+CD81+} was determined in a healthy control group without known CVD (n=9), compensated heart failure (CHF, n=16) and decompensated heart failure (DHF, n=14). Statistical significance is indicated using a non-parametric t-test.

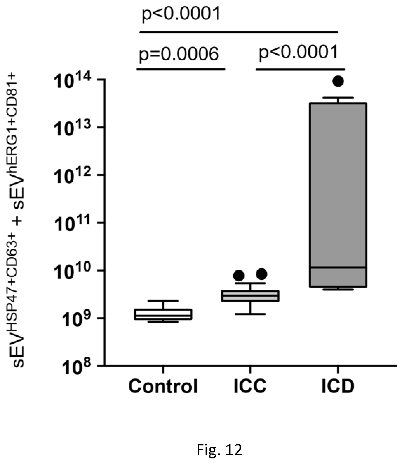

Figure 12. Reason for diagnosis, prognosis, and follow-up of decompensated heart failure. The relationship consisting of the sum of the concentrations of EVs with HSP47-CD63 and EVs with hERG1-CD81 (EV ^{HSP47+CD63+} + EV ^{hERG1+CD81+}) in patients under study was evaluated. From 0.1 mL of platelet-free plasma, the concentration of a healthy control group (with no known cardiovascular pathology) (n=9), compensated heart failure (CHF, n=16) and decompensated heart failure (DHF, n=14) was determined. Statistical significance is indicated using a non-parametric t-test.

### DESCRIPTION OF THE INVENTION

The present invention provides a method of diagnosis in blood of heart disease based on the identification, quantification and characterization of extracellular vesicles that have as biomarkers the potassium channel hERG1 and the chaperone Hsp47 on the surface of extracellular vesicles. The method according to the invention makes it possible to support the current diagnostic processes of this type of disease

The invention solves the problem of early identification of patients who develop heart disease, especially three patient segments:
i. Patients who are admitted to the emergency room with chest pain (angina) without current diagnostic systems being able to establish that it is a heart problem.
ii. Patients of the stress test, who have an inconclusive diagnosis of cardiac ischemia, since this test produces about 30% of false negatives.
iii. Patients with chronic heart failure who evolve to a decompensated state in which they do not respond to pharmacological therapy.

The basis of the invention lies in the fact that, during cardiovascular disease, heart cells release extracellular vesicles (EVs) that contain markers of the heart, fibrosis, or other conditions, where a specific selection of these markers is used in the diagnosis and prognosis of CVD with sufficient sensitivity and specificity for the diagnosis of this disease.

The KCNH2 gene encodes an ion channel involved in the fast component of I_{Kr}, the voltage-dependent potassium channel subunit-α KV11.1 (also called the human gene related to the ether-à-go-go 1, hERG1 gene) is expressed in the heart, regulating the cardiac action potential. hERG1 is responsible for the transport of potassium, responsible for the electrical activity of the heart and, therefore, the contraction of heart cells that determines the proper function of this organ. Mutations in the KCNH2 gene lead to the development of long QT syndrome (LQTS), a condition of cardiac repolarization that predisposes affected individuals to arrhythmia, that is, irregular rapid heartbeats that can cause fainting and sudden death.

A protein of the HSP family whose function is involved in the biosynthesis and correct folding of collagen is HSP47. Hsp47 is a stress-inducible collagen-specific molecular chaperone involved in the processing and secretion of procollagen. This collagen chaperone is expressed in the heart during episodes of stress and damage to the heart that could be associated with fibrosis or be a prelude to fibrosis and that establishes pathophysiological states.

During the development of the present invention, the expression of hERG1 and Hsp47 in extracellular vesicles, anchored to the vesicular membrane, was identified for the first time, and it was established that the quantification of total EV, EV-hERG1 (sEVs with the presence of hERG1) and EV-Hsp47 (sEVs with the presence of Hsp47) allows for discriminating between positive and negative cardiac ischemia, and also between compensated and decompensated heart failure.

Therefore, the data presented allowed the establishment of biomarkers of these two types of CVD, obtaining a sensitive and accurate method, based on the detection and quantification of hERG1 and Hsp47 in the fraction of extracellular vesicles (EVs) released by cardiac cells during episodes of heart damage.

Additionally, extracellular vesicles containing such proteins (EV-hERG1 and EV-Hsp47) were characterized in a blood sample, classifying them according to the size of the vesicles that present the protein markers.

Based on this information, a correlation was determined between the plasma levels of these extracellular vesicles and heart disease. In this way, the invention provides a diagnostic method for cardiovascular pathologies that considers the presence and concentrations of hERG1, Hsp47 and the size of extracellular vesicles in peripheral blood samples.

The method according to the present invention comprises the following steps:
- generating a platelet-free plasma (PFP) from a peripheral blood sample
- determining the concentration and size of total small extracellular vesicles (sEVs) present in plasma
- identifying and determining the concentration of sEVs with the presence of hERG1 (EV-hERG1)
- identifying and determining the concentration of sEVs with the presence of Hsp47 (EV-Hsp47)
- establishing the diagnosis of the heart condition.

Optionally, the size or size range of the sEVs associated with the presence of each protein is determined, that is, the size or size range of the EV-hERG1 and the EV-Hsp47 are determined.

The individual particle size and actual particle size distribution can be determined with any methodology available in the art, including transmission electron microscopy (TEM), tunable resistive pulse sensor (TRPS), dynamic light scattering (DLS), nanoparticle tracking analysis (NTA), tunable resistive pulse sensor (TRPS), or other methodologies.

In a realization of the invention, the concentration and size of extracellular vesicles present in the plasma of patient samples is performed using the nanoparticle tracking analysis (NTA) methodology, for example, on a NanoSight device. This technique allows particles from 50 to 1,000 nm to be visualized and quantified in real time. The NTA methodology can even be combined with fluorescence (NTA-FL) using fluorescent antibodies to characterize extracellular vesicles with specific proteins in the vesicle membrane. Therefore, the NTA-FL methodology allows for quantifying of sEVs with specific markers, such as VE-hERG1 and VE-Hsp47.

In a realization of the invention, after the PFP has been extracted, the sEVs are concentrated, for example, by ultracentrifugation, size exclusion chromatgraphography, immunocapture, or others. Extracellular vesicles containing the hERG1 protein are quantified using specific antibodies that recognize the extracellular domain of the hERG1 protein. In a realization of the invention, these antibodies recognize the SEQ sequence. ID No 1 (⁴³⁰AFLLKETEEGPAPATE⁴⁴⁵).

Extracellular vesicles containing the Hsp47 protein are quantified using specific antibodies that recognize a region of the Hsp47 protein. In a realization of the invention, these antibodies recognize the SEQ sequence. ID No 2 (¹⁶⁹ALQSINEWAAQTTDGKLPEV TKDVERTD¹⁹⁶) and is located in the extravesicular region

The assay allows for establishing a range in concentrations of the analytes that will allow a specialist to establish the risk of cardiovascular disease. The ranges considered in each case are as follows:
∘ positive cardiac ischemia: (i) total EV concentration greater than 1x10¹⁰ EV/mL of blood plasma. (ii) concentration of EV-hERG1 between 60 and 120 nm greater than 1x10⁷. (iii) concentration of EV-Hsp47 between 120 and 300 nm greater than 1x10⁴ EV/mL of blood plasma
∘ negative cardiac ischemia: (i) total EV concentration less than 1x10¹⁰ EV/mL of blood plasma. (ii) concentration of EV-hERG1 between 60 and 120 nm less than 1x10⁷ EV/mL of blood plasma (iii) concentration of EV-Hsp47 between 120 and 300 nm less than 1x10⁴ EV/mL of blood plasma
∘ compensated heart failure (CHF): (i) total EV concentration less than 1x10¹¹ EV/mL of blood plasma. (ii) concentration of EV-hERG1 greater than 1x10¹⁰ EV/mL of blood plasma. (iii) concentration of EV-Hsp47 greater than 1x10¹⁰ EV/mL of blood plasma
∘ decompensated heart failure (DHF): Can be defined when the EV-Hsp47/EV-hERG1 ratio is less than 1.

Therefore, using a peripheral blood sample obtained by standard clinical laboratory methods, it is possible to characterize and quantify total extracellular vesicles and those containing the hERG1 and Hsp47 proteins directly from blood plasma. The method uses immunoassay platforms currently available in clinical laboratories around the world, such as the ELISA (*Enzyme-Linked Immunosorbent Assay*) technique.

### Method of quantifying EV with the proteins of interest

In this phase, the method selected for the quantification of proteins in sEVs corresponds to immunoassay methods regularly used in clinical laboratories around the world, such as the ELISA technique. Because there is no commercially available ELISA product on the market for the quantification of hERG1 or HSP47 proteins present in extracellular vesicles from a blood sample, the strategy for the present invention was to use ELISA prototypes with technical, analytical and validation steps developed by the inventors' research team.
a. ELISA EV^{Hsp47+CD63+} prototype. This prototype has the ability to quantify extracellular vesicles containing the HSP47 protein and the CD63 protein (sEVs marker) called EV ^{Hsp47+CD63+}. The ELISA EV^{Hsp47+CD63+} prototype uses a positive control to elaborate a standard curve in order to establish the association between the signal originated by an ELISA reader equipment (absorbance at 450nm) and the concentration of EV ^{Hsp47+CD63+} (Figure 8). ELISA reader equipment is validated for regular use in clinical laboratories.
b. ELISA EV ^{hERG1+CD81+} prototype. This prototype has the ability to quantify extracellular vesicles containing the hERG1 protein and the CD81 protein (sEVs marker) called EV ^{hERG1+CD81+}. The ELISA EV ^{hERG1+CD81+} prototype uses a positive control for the elaboration of a standard curve to determine the association between the signal originated by the ELISA reader equipment (absorbance at 450nm) and the concentration of EV ^{hERG1+CD81+} (Figure 9). As mentioned above, ELISA reader equipment is validated for regular use in clinical laboratories.
c. Relationship of the sum of heart failure biomarkers.

The relationship between the concentration of EVs containing HSP47 and CD63 proteins and EVs containing hERG1 and CD81 proteins was established.

The ratio of the sum of EV ^{HSP47+CD63+} + EV ^{hERG1+CD81+} shows values that allow for establishing a relationship for the diagnosis of decompensated heart failure. In this way, values of 8x10⁸ to 2x10⁹ extracellular vesicles per mL were determined for healthy patients; 1x10⁹ to 8.4x10⁹ extracellular vesicles per mL for patients with compensated heart failure and 4x10⁹ to 1x10¹⁴ extracellular vesicles per mL for patients with decompensated heart failure.

In this way, it is possible to carry out the validation stage of the biomarker data with two ELISA prototypes developed by the inventors.

The analyses successfully showed the scientific-technical information for the development of several products based on these biomarkers of cardiovascular diseases.

In particular, it was validated that:
1. The EV ^{HSP47+CD63+} biomarker is useful for assessing the diagnosis of decompensated heart failure. This biomarker presents values in the order of 7x10⁸ to 2x10⁹ extracellular vesicles per mL for healthy patients. 1x10⁹ to 5x10⁹ extracellular vesicles per mL for patients with compensated heart failure, and 4x10¹⁰ to 1x10¹⁴ extracellular vesicles per mL for patients with decompensated heart failure.
2. The EV ^{hERG1+CD81+} biomarker is suitable for assessing the evolution of decompensated heart failure. This biomarker has values in the range of 6x10⁶ to 1x10⁸ extracellular vesicles per mL for healthy patients, 1.2x10⁸ to 6.4x10⁹ extracellular vesicles per mL for patients with compensated heart failure, 1x10⁷ to 2.4x10¹⁰ extracellular vesicles per mL for patients with decompensated heart failure.
3. The sum of EV ^{HSP47+CD63+} + EV^{hERG1+CD81+} can be used for the diagnosis, prognosis, and follow-up of decompensated heart failure. As indicated above, the values are in the order of 8x10⁸ to 2x10⁹ extracellular vesicles per mL for healthy patients, 1x10⁹ to 8.4x10⁹ extracellular vesicles per mL for patients with compensated heart failure, and 4x10⁹ to 1x10¹⁴ extracellular vesicles per mL for patients with decompensated heart failure.

The ranges have been considered standard deviation, so there is a slight overlap in the contiguous limits, which can be discerned based on the other analyses.

Once the diagnosis of heart disease has been determined, the medical professional can determine the appropriate treatment for each condition, including, for example, the use of medications, cardiac rehabilitation, devices (defibrillators, pacemakers, ventricular assist device, artificial heart, etc.), cardiac procedures (cardioversion, percutaneous coronary intervention, stent, or *stent*, catheter ablation, bypass surgery, transcatheter aortic valve replacement, cardiac surgery, etc.) or heart transplant.

### EXAMPLES

### Patient recruitment and sampling

A total of 40 patients undergoing the stress test were recruited, considering people over 18 years of age who signed informed consent. 20 patients with a positive diagnosis of cardiac ischemia and 20 patients with a negative diagnosis of cardiac ischemia were obtained. Blood samples were taken before the cardiac stress test and 15 minutes after the cardiac stress test. Additionally, a total of 20 patients with chronic heart failure were recruited, considering people over or equal to 18 years of age, who signed the informed consent.

Blood samples were collected using EDTA. Plasma was obtained by centrifugation of fresh blood for 15 min, 1500 x g, 4°C (HeraCell, Thermo) to produce a platelet-free plasma (PFP). The PFP was transferred to cryotubes and stored at -80°C.

### Example 1: Characterization of hERG1 and Hsp47 in extracellular vesicles using Nanoparticle Tracking Analysis

Using a primary culture of cardiomyocyte cells, the effect of hypoxia on EV release on a time-course curve was studied. EV levels were measured using nanoparticle tracking (NTA) analysis on the *NanoSight* instrument directly from culture media (no ultracentrifugation). The results showed that from 15 min exposed to 1% oxygen, cardiomyocytes significantly increased the release of EVs compared to 21% oxygen, peaking at 30min and experiencing a significant reduction towards 8 hrs of hypoxia, with 8 hrs of hypoxia still exceeding 21% oxygen (Figure 1A). Therefore, cardiomyocytes release EV by hypoxia as early as 15 min into oxygen deprivation.

Taking this information into account, the concentration of EV in human blood samples from patients was studied 15 minutes after finishing the test. The stress test makes the heart pump harder and faster and therefore requires more blood perfusion to the heart and therefore this test can determine the existence of problems with blood flow within the heart. Thus, if the heart does not receive adequate blood due to the patient's health condition, cardiac ischemia occurs and is diagnosed during the stress test. Therefore, if the patient has their coronary arteries blocked, the Stress Test test induces a transient period of cardiac ischemia where the cardiologist can diagnose this condition in the vast majority of cases. In this scenario, the concentration of sEVs was studied in blood samples from healthy individuals at rest (without known cardiac pathologies), and participants of the stress test with a diagnosis of negative cardiac ischemia and positive cardiac ischemia. Healthy controls and ischemic negative participants showed a similar concentration of EV in the blood. Interestingly, participants with positive cardiac ischemia had a significantly higher concentration of EV than negative cardiac ischemia after 15 min of the stress test (Figure 1B), suggesting that at 15 minutes after cardiac ischemia elicited by the stress test, EV increases in blood. One of the participants with a negative diagnosis of cardiac ischemia in the stress test had elevated EV in the blood (Figure 2B, data shown with an arrow), this participant has a history of drug abuse, a condition associated with alterations in the cardiac blood supply, which could potentially be associated with elevated EV in the blood.

Our research has shown for the first time that extracellular vesicles present in human blood possess the expression of the proteins hERG1 and Hsp47 in the vesicular membrane (Figure 2). To analyze the concentration of EV with positive presence of hERG1 or Hsp47 in blood samples, anti-hERG1 or anti-Hsp47 antibodies, fluorophore-conjugated secondary antibody, and determination of NTA-associated fluorescence were used. The first step was to establish the concentration of EVs from each blood sample. Then, an amount of 1x10⁹ EV was used as the standard for EV at NTA fluorescence. In this way, a very small volume of PFP (5-20 µL) was used to obtain the total amount of 1x10⁹ EV for the assay. PFP samples were diluted to 500µL with DPBS and incubated for 1 hour with the respective primary antibody (anti-hERG1 or anti-Hsp47) and then another 1 hour with the secondary antibody conjugated with Alexa Fluor 532. The antibodies were not washed, considering that the actual size of an antibody molecule is about 10 nm, smaller than the EVs of interest (50-200nm), so they are not considered in the analysis. Consequently, using small PFP samples belonging to cardiac ischemic positive and negative participants, EV was measured with hERG-1 (EV-hERG1) or Hsp47 (EV-Hsp47). The concentration of EV-hERG1 was detected in different sizes of nanoparticles (Figure 3A). Interestingly, the group classified as cardiac ischemia had a significantly higher average concentration of EV-hERG1 than participants with negative cardiac ischemia (Figure 3A). In addition, EV-hERG1 between 60 and 120 nm is more expressed in the positive cardiac ischemia group.

Using another group of participants, the concentration of EV-Hsp47 in PFP was studied. The group classified as cardiac ischemia had a significantly higher average concentration of EV-Hsp47 than participants with negative cardiac ischemia (Figure 3B). In addition, EV-Hsp47 between 120 and 300 nm is more expressed in the positive cardiac ischemia group.

### Example 2: h-ERG1 and Hsp47 are present in EV obtained from blood samples from participants with heart failure.

EV-hERG1 and EV-Hsp47 were characterized in participants' blood samples corresponding to compensated heart failure (CHF) and decompensated heart failure (DHF). The EVs were enriched by ultracentrifugation from PFPs from each participant to use the sediment to characterize hERG1, Hsp47, and CD9 (small extracellular vesicle marker) proteins using *Western blot* and flow cytometer. All three proteins analyzed by *Western blot were* detected in all participants diagnosed with heart failure (Figure 4).

Another technique to characterize the presence of sEVs is flow cytometry, by binding sEVs to 1-micrometer-sized beads and characterizing vesicular membrane proteins. Using the ultracentrifuged fraction of sEVs and beads, anti-hERG1 or anti-Hsp47 antibodies were used to characterize the presence of these proteins in the sEVs of the patients under study.

Positive signals for the hERG-1 and Hsp47 proteins were found in the analyzed sEVs of all participants studied by flow cytometry (Figure 5A and 5B). Therefore, both proteins are detectable in the EVs of patients with compensated and decompensated heart failure (Figure 5). The EV-Hsp47/EV-hERG1 ratio was determined in each of the patients and, interestingly, a significantly lower ratio was found in patients with DHF compared to CHF (Figure 5C). It was established that patients with decompensated heart failure have lower levels of sEVs with the fibrosis marker Hsp47 in relation to heart markers (hERG1).

### Example 3: The abundance of extracellular vesicles with the proteins hERG-1 and Hsp47 are decreased in the blood of patients with decompensated heart failure.

Using NTA fluorescence, participants with CHF (n = 10) and DHF (n = 10) were seen to have detectable levels of EV-hERG1 and EV-Hsp47 in their blood plasma. In addition, patients with DHF were found to have significantly lower concentration of sEVs than patients with CHF (Figure 6A). The concentration of EV-hERG1 decreased in participants with DHF compared to participants with CHF.

The data established that, on average, the concentration of EV-hERG1 was 10 times lower in DHF than in CHF (Figure 6B), where the values were in the order of 10⁹ to 10¹⁰ for DHF and 10¹⁰ to 10¹¹ for CHF. The quantification of EVs with the fibrosis-associated protein (Hsp47) indicates that patients with DHF also had a lower concentration of EV-Hsp47 than patients with CHF (Figure 6C), where the values were in the order of 10⁹ to 10¹⁰ for DHF and 10¹⁰ to 10¹¹ for CHF.

EV size distribution analysis showed that between 50 and 500 nm a similar size was found for total EV, EV-hERG1, and EV-Hsp74 (Figure 6D-F). In the case of decompensated heart failure, a very significant reduction was obtained in total EV, EV-hERG1 and EV-Hsp74 in the size range of 50-300 nm, where the values were in the order of 10⁷ for DHF and 10⁸ to 10⁹ for CHF, especially in the EV-Hsp74 with values in the order of 10⁹ for CHF, being two orders of magnitude higher than the compensated group (Figure 6G-I).

The correlation between total EV and EV-hERG1 concentrations and total EV and EV-Hsp47 concentrations was analyzed for each group of patients. The results allow us to establish that compensated heart failure behaved differently from participants with decompensated heart failure, from which a relationship can be deduced to differentiate these two types of patients (Figure 7). Thus, in this way, patients with compensated heart failure have larger vesicles with the proteins hERG1 and Hsp47 (Figure 7A), compared to those with decompensated heart failure.

### Example 4: Validation of biomarkers of cardiovascular diseases.

The validation of the presence of the biomarkers hERG1 and HSP47 in extracellular vesicles present in the blood of patients with cardiovascular diseases was carried out with a cohort of 60 patients, consisting of controls without ischemic heart pathology and with a diagnosis of heart failure (Table 1).

Two types of patients were recruited from the group of patients with heart failure:
(i) patients who respond favorably to cardiology therapy, known as compensated heart failure, CHF (16 patients), and
(ii) patients who do not respond to treatment and worsen their heart condition, known as decompensated heart failure, DHF (14 patients).

Patients with DHF are the most serious, since they decompensate in their cardiac function and have several episodes of emergency hospitalization during the year.

**Table 1. Description of the patients analyzed**

| | Control (n=30) | CHF (n=16) | DHF (n=14) |
|---|---|---|---|
| Gender (male, %) | 76.67 | 62.50 | 57.14 |
| Age (years, mean±SD) | 55.80 ± 12.75 | 66.13 ± 13,73 | 62.93 ± 17.83 |
| SBP (mmHG, media±DE) | N/A | 125.81 ± 22.00 | 134.28 ± 34.96 |
| DBP (mmHG, media±DE) | N/A | 68.25 ± 11.56 | 82.71 ± 19.24 |
| HT (%) | 63.33 | 56.25 | 78.57 |
| Dyslipidemia (%) | 30.00 | 6.25 | 21.43 |
| DM (%) | 26.67 | 6.25 | 21.43 |
| CKD (%) | 3.33 | 0 | 35.71 |
| Smoker (%) | 30.00 | 0 | 14.29 |
| Implantable cardioverter-defibrillator (ICD) | 0 | 31.25 | 21.43 |

| | | | |
|---|---|---|---|
| SBP, systolic blood pressure. DBP, diastolic blood pressure. HT, hypertension. DM, diabetes mellitus. | | | |

### a. Validation of HSP47/CD63 biomarkers in blood EVs using the ELISA EV ^{Hsp47+CD63+} prototype.

From 0.1mL of platelet-free plasma (produced by a clinical laboratory method that takes 10 minutes) the concentration of extracellular vesicles containing the biomarker HSP47 associated with the vesicular marker CD63 was determined using the ELISA EV^{HSP47+CD63+} kit. The results show that the control group has on average about 1x10⁹ extracellular vesicles per mL with the HSP47/CD63 markers (EV^{HSP47+CD63+}) and the patients with CHF have on average a slight and significantly higher value (p=0.0139, Figure 10). Interestingly, patients with the most severe condition of heart failure (DHF) have a statistically significant increase in plasma EV^{HSP47+CD63+} concentration (which on average is 10 times higher than in healthy controls, Figure 10). In addition, there is a group of patients who have four orders of magnitude (10,000 times) higher concentration of EV^{HSP47+CD63+} in blood than the average of the healthy control group. These data suggest that the quantification of EV^{HSP47+CD63+} constitutes a biomarker of decompensation in patients with cardian insufficiency and therefore a biomarker of prognosis and follow-up of the pathology.

### b. Validation of hERG1-CD81 biomarkers in blood EVs using the ELISA EV^{Hsp47+CD63+} prototype.

From 0.1mL of platelet-free plasma (produced by a clinical laboratory method that takes 10 minutes) the concentration of extracellular vesicles containing the hERG1 biomarker associated with the vesicular marker CD81 was determined using the ELISA EV^{hERG1+CD81+} kit. The results show that the healthy control group has on average about 3.3x10⁷ extracellular vesicles per mL with the markers hERG1-CD81 (EV^{hERG1+CD81+}) and patients with CHF have a significantly higher average value (5.5x10⁸, p=0.0003, Figure 11). Interestingly, patients with the most severe condition of heart failure (DHF) have a statistically significant higher concentration of plasma EV^{hERG1+CD81+} compared to controls (6x10⁸, p<0.0078, Figure 11). This data suggest that the quantification of EV^{hERG1+C81+} constitutes a biomarker of insufficiency.

### c. Relationship of heart failure biomarkers.

The relationship of biomarkers of interest for the diagnosis of compensated and decompensated heart failure was evaluated in the study group. In this way, the sum of the concentration of EV containing the HSP47 and CD63 proteins and the EV containing the hERG1 and CD81 proteins was established.

In this way, the sum (EV^{HSP47+CD63+} + EV^{hERG1+CD81+}) was evaluated in each patient (Figure 12). The data allow us to establish that on average the sum of the sEVs in healthy control patients was 1x10⁹, 3x10⁹ in patients with CHF and 2x10¹⁰ in patients with DHF (Figure 12). The maximum value observed in DHF patients was 9.3x10¹³. This data suggest that this relationship is strongly related to DHF and therefore constitutes a biomarker for the diagnosis, prognosis, and follow-up of decompensated heart failure (Figure 12).

## Claims

1. An *in vitro diagnostic method* of cardiovascular disease, **CHARACTERIZED in that** it includes the steps of:
a) Removing platelet-free plasma (PFP) from a peripheral blood sample
b) Concentrating the total small extracellular vesicles (sEVs) present in plasma
c) Determining the total concentration and size of sEVs
d) identifying and determining the concentration of sEVs with the presence of the potassium channel protein hERG1 (VE-hERG1),
e) identifying and determining the concentration of sEVs with the presence of the Hsp47 protein (VE-Hsp47),
f) Establishing the diagnosis of the heart condition.

2. The diagnostic method according to claim 1, **CHARACTERIZED in that** the size or range of size of sEVs associated with the presence of each protein is optionally determined.

3. The diagnostic method according to claim 2, **CHARACTERIZED in that** the size of the sEVs is determined with a methodology selected from between transmission electron microscopy (TEM), tunable resistive pulse sensor (TRPS), dynamic light scattering (DLS), nanoparticle tracking analysis (NTA), tunable resistive pulse sensor (TRPS), or other methodology available in the state of the art.

4. The diagnostic method according to claim 1, **CHARACTERIZED in that** extracellular vesicles containing the hERG1 protein are quantified by an ELISA EV^{hERG1+CD81+} method.

5. The diagnostic method according to claim 4, **CHARACTERIZED in that** the EV^{hERG1+CD81+} ratio has values in the order of:
• 6x10⁶ to 1x10⁸extracellular vesicles per mL for healthy patients
• 1.2x10⁸ to 6.4x10⁹ extracellular vesicles per mL for patients with compensated heart failure,
• 1x10⁷ to 2.4x10¹⁰ extracellular vesicles per mL for patients with decompensated heart failure.

6. The diagnostic method according to claim 1, CHARACTERIZED because extracellular vesicles containing the Hsp47 protein are quantified by an ELISA EV^{Hsp47+CD63+} method.

7. The diagnostic method according to claim 6, **CHARACTERIZED in that** the EV^{Hsp47+CD63+} ratio has values in the order of:
• 7x10⁸ to 2x10⁹ extracellular vesicles per mL for healthy patients;
• 1x10⁹ to 5x10⁹ extracellular vesicles per mL for patients with compensated heart failure; or
• 4x10¹⁰ to 1x10¹⁴ extracellular vesicles per mL for patients with decompensated heart failure.

8. The diagnostic method according to claim 1, **CHARACTERIZED in that** the diagnosed cardiac condition is selected from positive cardiac ischemia, compensated heart failure, or decompensated heart failure.

9. The diagnostic method according to claim 1, **CHARACTERIZED in that** after extracting platelet-free plasma EVs are concentrated by ultracentrifugation, exclusion chromatography, or immunopurification.

10. The diagnostic method according to claim 1, **CHARACTERIZED in that** the ratio consisting of the sum of the concentration of EV contained in the proteins HSP47 and CD63 (EV^{HSP47+CD63+} + EV^{hERG1+CD81+}) presents values in the order of:
a. 8x10⁸ to 2x10⁹ extracellular vesicles per mL for healthy patients;
b. 1x10⁹ to 8.4x10⁹ extracellular vesicles per mL for patients with compensated heart failure; or
c. 4x10⁹ to 1x10¹⁴ extracellular vesicles per mL for patients with decompensated heart failure.
